# EUROPÄISCHE PATENTSCHRIFT

(11) **EP 3 555 038 B1**
(45) Veröffentlichungstag und Bekanntmachung des Hinweises auf die Patenterteilung: **30.09.2020**
(21) Anmeldenummer: 17832758.1
(22) Anmeldetag: 13.12.2017
(51) Int. Cl.: C07C 67/54, C07C 69/54

(54) **VERFAHREN ZUR DESTILLATIVEN GEWINNUNG VON REINEM CYCLOHEXYL(METH)ACRYLAT**
METHOD FOR THE RECOVERY OF PURE CYCLOHEXYL (METH)ACRYLATE BY MEANS OF DISTILLATION
PROCÉDÉ DE PRODUCTION PAR DISTILLATION DE CYCLOHEXYL(MÉTH)ACRYLATE PUR

(30) Priorität: 13.12.2016 EP 16203787
(43) Veröffentlichungstag der Anmeldung: 23.10.2019
(73) Patentinhaber: BASF SE, 67056 Ludwigshafen am Rhein (DE)
(72) Erfinder: LANG, Ortmund, 67056 Ludwigshafen (DE); KLIPFEL, Nadja Isabelle Desiree, 1950 Sion (CH); JANSSEN, Nicole, 67056 Ludwigshafen (DE); POX, Roland, 67056 Ludwigshafen (DE)
(74) Vertreter: BASF IP Association
(86) Internationale Anmeldenummer: PCT/EP2017/082695
(87) Internationale Veröffentlichungsnummer: WO 2018/109040

(56) Entgegenhaltungen:
- EP-A1- 1 853 546
- WO-A1-03/045894

## Beschreibung

Die vorliegende Erfindung betrifft ein Verfahren zur destillativen Gewinnung von reinem Cyclohexyl(meth)acrylat aus einem Gemisch enthaltend Cyclohexyl(meth)acrylat, Cyclohexanol und Schleppmittel.

Der Begriff (Meth)acrylsäure bezeichnet in bekannter Weise Acrylsäure und/oder Methacrylsäure. Analog steht Cyclohexyl(meth)acrylat für Cyclohexylacrylat und/oder Cyclohexylmethacrylat.

Cyclohexyl(meth)acrylat ist ein Spezialmonomer für den Lack- und Anstrichsektor. Wichtige Anwendungen sind beispielsweise top clear coats für Automobillacke, Harze für lösemittelhaltige und -freie Lacke, witterungsbeständige Außenanstrichdispersionen sowie Klebstoffe.

Bekannte Verfahren zur großtechnischen Herstellung von Cyclohexyl(meth)acrylat basieren überwiegend auf der Umesterung eines (Meth)acrylats, insbesondere Methyl(meth)acrylat mit Cyclohexanol in Gegenwart eines Katalysators. Nachteilig an den Umesterungsverfahren zur Herstellung von Cyclohexyl(meth)acrylat ist, dass es sich aus kinetischen Gründen ungünstig darstellt, mit entsprechend niedrigen Raum-Zeit-Ausbeuten.

Ein Verfahren zur Veresterung von (Meth)acrylsäure mit Cyclohexanol ist in der EP 1 853 546 A1 (WO 2006/087297 A1), BASF AG, beschrieben.

Nach diesem Verfahren werden nach der Veresterung zunächst die Leichtsieder (über Kopf) und anschließend die Schwersieder (über Sumpf) aus dem Rohprodukt destillativ abgetrennt. In der Leichtsieder-Destillation wird zunächst das Schleppmittel über Kopf abdestilliert ("Verfahrensstufe D"). Anschließend wird in einer Kolonne der Alkohol über Kopf und der Zielester über den Sumpf abgetrennt ("Verfahrensstufe E"). Die Stufen "D" und "E" können auch in einer gemeinsamen Destillationseinheit durchgeführt werden.

Der Destillatstrom, der neben dem abgetrennten Alkohol in der Regel noch relativ große Mengen an Zielester enthält, wird zur Veresterungsstufe zurückgeführt (vgl. das Schema 1/1 in der WO 06/087297).

Die Rückführung von Produkt führt nicht nur zu einer Verkleinerung der Gesamtkapazität der Anlage, sondern beeinflusst auch den Veresterungsumsatz in einer negativen Art und Weise. Insbesondere wenn als Sumpfverdampfer in der "Verfahrensstufe A" ein Fallfilmverdampfer in Gegenstromfahrweise betrieben wird, kann es aufgrund des verwendeten Verdampfertyps in der Anlage zu Qualitätsproblemen mit dem Wertprodukt (Zielester) kommen. Außerdem wird die Gesamtkapazität der Anlage durch die Gegenstromfahrweise begrenzt. Bei den gegebenen Betriebsbedingungen kann es zum Aufstauen des nach unten abfließenden Flüssigkeitsgemisches durch das nach oben strömende Dampfgemisch kommen. Bei entsprechend hoher angestauter Flüssigkeitssäule kommt es aufgrund des hydrostatischen Druckes zu einem schlagartigen Ablaufen der Flüssigkeit und danach wieder zu einem Anstauen. Dies führt zu einem Pulsieren in der Kolonne. Eine stabile Betriebsweise der Destillationskolonne kann durch die Pulsation bei dieser Gegenstromfahrweise im Fallfilmverdampfer stark eingeschränkt sein.

Die Abtrennung des Alkohols vom Zielester ist bei einer weiteren Kapazitätserweiterung ggf. nicht mehr möglich, der Alkohol gelangt in den Sumpfbereich der Kolonne und die Produktqualität kann evtl. nicht mehr gewährleistet werden.

Das beschriebene Verfahren hat also insbesondere den Nachteil, dass durch die Rückführung des Destillats zur Veresterung relativ große Mengen an Zielester erneut der Veresterungsblase zugeführt werden. Die thermische Belastung welcher das Produkt erneut ausgesetzt ist, erhöht die Bildung von Nebenkomponenten. Dadurch wird die Produktausbeute erniedrigt und die Qualität verringert. Durch die mehrfache Destillation des Zielesters ist der Energieaufwand besonders hoch. Ein weiterer Nachteil ist das Betreiben des Fallfilmverdampfers in Gegenstromfahrweise. Instabilitäten beim Betreiben der Kolonne, Fouling und eine damit verbundene kürzere Laufzeit des Verdampfers sind die Folge davon.

Ullmann's Encyclopedia of Industrial Chemistry, Vol. A 1, Kapitel 'Acrylic Acid and Derivatives', Verlag VCH 1985, beschreibt auf den Seiten 161 bis 176 die destillative Aufarbeitung von höheren Alkylacrylaten aus einer Veresterungsreaktion (Fig. 5, Seite 168). Demgemäß wird in einer Kolonne f das Lösungsmittel über Kopf abgetrennt, anschließend in einer Seitenzulaufkolonne g der unumgesetzte Alkohol abdestilliert und schließlich der Produktester in der Kolonne h reindestilliert.

Der vorliegenden Erfindung lag die Aufgabe zugrunde, ein verbessertes Verfahren zur Herstellung von Cyclohexyl(meth)acrylat aus (Meth)acrylsäure und Cyclohexanol zur Verfügung zu stellen, das, bei Einhaltung der geforderten Spezifikationen für das Zielprodukt die Nachteile des Standes der Technik nicht aufweist, und insbesondere einen qualitativ hochwertigen Zielester in einem energetisch günstigen Verfahren zur Verfügung stellt.

Demgemäß wurde ein Verfahren der destillativen Gewinnung von reinem Cyclohexyl(meth)-acrylat aus einem Gemisch enthaltend Cyclohexyl(meth)acrylat, Cyclohexanol und Schleppmittel gefunden, welches dadurch gekennzeichnet ist, dass man das Gemisch einer Rektifikationskolonne, mit Sumpfverdampfer, trennwirksamen Einbauten und einem Abtriebs- und einem Verstärkungsteil, als Seitenzulauf zuführt, wobei die Kolonne 4 bis 50 theoretische Trennstufen aufweist, der seitliche Zulauf im Bereich beginnend mindestens eine Trennstufe oberhalb der untersten und endend mindestens eine Trennstufe unterhalb der obersten Stufe erfolgt, der Druck am Kopf der Kolonne im Bereich von 10 mbar bis 5 bar liegt und das Rücklaufverhältnis 1 : 0,2 bis 1 : 10 beträgt, und den Sumpfaustrag einer weiteren Destillationseinheit zuführt, in der das Cyclohexyl(meth)acrylat über Kopf oder einen Seitenablauf abgetrennt wird.

Das Verfahren wird bevorzugt kontinuierlich ausgeführt.

Bei dem Schleppmittel (für Wasser) handelt es sich bevorzugt um Cyclohexan.

Im erfindungsgemäßen Verfahren wird der unumgesetzte Alkohol, also das Cyclohexanol, und weitere Leichtersieder als das Wertprodukt in einem spezifischen Schritt in einer Kolonne mit seitlichem Zulauf (Seitenzulaufkolonne) über Kopf abdestilliert. Das Wertprodukt, also der (Meth)acrylsäurecyclohexylester, wird über einen Sumpfabzug einer nachfolgenden Destillationseinheit zugeführt.

Es wurde überraschend gefunden, dass durch die spezielle Ausgestaltung und speziellen Bedingungen der Seitenzulaufkolonne bei der destillativen Abtrennung der Leichtersieder, nämlich insbesondere Cyclohexanol und Schleppmittel, der Zielester, also der (Meth)acrylsäure-cyclohexylester, sehr stark im Destillat abgereichert werden kann.

Dadurch wird gleichzeitig der Rückstrom in die erste Veresterungsstufe stark verringert und zusätzlich wird die Kapazität einer Anlage zur Herstellung von Cyclohexyl(meth)acrylat deutlich erhöht sowie der Umsatz der Reaktion durch den zusätzlichen freien Reaktionsraum gesteigert.

Ebenfalls resultiert durch die erfindungsgemäße Verfahrensweise, dass durch die geringeren Rückführströme eine Gesamtanlage zur Herstellung von Cyclohexyl(meth)acrylat mit einer geringeren Energiemenge betrieben werden kann. Weiterhin kommt es durch die geringere thermische Belastung des Zielesters zu einer Verringerung der Bildung von unerwünschten Nebenkomponenten durch Zersetzung des Zielesters.

Unter üblichen Destillationsbedingungen kommt es zu Zersetzungsreaktionen, zu Verfärbungen und zu einer unzureichenden Abtrennung der Nebenkomponenten, so dass die erforderliche Reinheit des Cyclohexyl(meth)acrylats für eine weitere Verwendung ggf. nicht ausreichend ist. Daher stellt die Aufreinigung der Cyclohexyl(meth)acrylat ein dauerhaftes Problem dar. Dies gilt insbesondere für Nebenkomponenten mit einem hohen Siedepunkt, da diese bei Bedingungen abgetrennt werden, bei denen eine Zersetzung und eine oft damit verbundene Produktschädigung nicht unterdrückt werden kann. Dementsprechend ist es äußerst schwierig, insbesondere das Wertprodukt ohne hohe Ausbeuteverluste in einer sehr hohen Reinheit zu erhalten.

Im hier beschriebenen Verfahren kann erfindungsgemäß durch den Einsatz von druckverlustarmen Kolonneneinbauten die Sumpftemperatur und damit die Nebenkomponentenbildung weiter reduziert werden kann.

Versuche zur Temperaturstabilität haben gezeigt, dass die Bildung von Nebenkomponenten, die für die Verschlechterung der Produktqualität des Zielesters verantwortlich sind, mit zunehmender Temperatur ansteigt (siehe unten Beispiel 2).

Durch eine geringere Sumpftemperatur wird erfindungsgemäß die thermische Belastung des Zielesters reduziert und damit die Produktqualität gesteigert sowie die Bildung von Fouling in den Verdampfern verringert. Das geringere Fouling in den Verdampfern führt zu einer Erhöhung der Laufzeit und dadurch zu einer Kapazitätserhöhung.

Durch die geringeren Rückführströme kann die Kapazität einer Gesamtanlage zur Herstellung von Cyclohexyl(meth)acrylat z.B. um ca. 15 % erhöht werden.

Als Rektifikationskolonne kommt bespielweise eine Füllkörperkolonne, Packungskolonne oder Bodenkolonne in Frage. Die Kolonne ist, wie in der Regel jede Destillationskolonne, mit einem Sumpfverdampfer und einem Kondensator am Kolonnenkopf ausgestattet.

Das Verfahren wird erfindungsgemäß in einer Rektifikationskolonne mit Abtriebsteil und Verstärkungsteil und einem Sumpfverdampfer durchgeführt. Bei dem Sumpfverdampfer handelt es sich besonders um einen Dünnschichtverdampfer, der bevozugt in Gegenstromfahrweise betrieben wird, ganz besonders einen Fallfilmverdampfer, der bevorzugt in Gleichstromfahrweise betrieben wird.

Im Verfahren der vorliegenden Erfindung wird in besonders vorteilhafter Weise die Verweilzeit im Sumpfverdampfer und dem zugehörigen Rohrleitungssystem auf 1 bis 60 Minuten, bevorzugt auf 10 bis 30 Minuten, begrenzt. Dadurch werden trotz der Polymerisationsfähigkeit des Gemisches ein störungsfreier Betrieb der Anlage, insbesondere nur geringfügige oder keine Verschmutzungen gewährleistet.

Ein bevorzugtes Merkmal des erfindungsgemäßen Destillationsverfahrens ist, dass vorzugsweise die Verdampfung bei einer spezifischen Wärmebelastung im Bereich von 1 kW/m² bis 100 kW/m², besonders bevorzugt im Bereich von 2 kW/m² bis 50 kW/m² und ganz besonders bevorzugt 5 kW/m² bis 30 kW/m² durchgeführt wird, wobei diese Angaben auf die zugeführte Wärme und die beheizte Verdampferoberfläche des Verdampfers bezogen sind. Das Überschreiten des oberen Wertes von 100 kW/m² führt, wie erfindungsgemäß erkannt wurde, aufgrund der hohen erforderlichen Temperaturdifferenz zwischen Produkt und Heizmedium zu erhöhtem Fouling auf der Verdampferoberfläche. Die Unterschreitung des unteren Wertes von 1 kW/m² führt zu einem unwirtschaftlichen Verfahren da bei gegebener Wärmemenge die beheizte Verdampferoberfläche des Verdampfers zu groß oder bei gegebener beheizter Verdampferoberfläche des Verdampfers die zu übertragende Wärmemengen zu klein werden.

In einer bevorzugten Verfahrensvariante wird das Flüssigkeitsrücklaufverhältnis (also Quotient aus Rücklaufmenge und der abgezogenen Destillatmenge) der Kolonne im Verhältnis von 1 : 0,2 bis 1 : 10 (also 5 bis 0,1), weiter bevorzugt im Verhältnis von 1 : 0,2 bis 1 : 1 (also 5 bis 1), geregelt. Dies erfolgt bevorzugt in der Weise, dass die Flüssigkeit nach dem Kondensator gesammelt und über eine Regelungs- oder Stellvorrichtung im genannten Verhältnis auf die Kolonne bzw. den Destillatbehälter aufgegeben wird. Dadurch wird ein niedrigerer Energieverbrauch gewährleistet.

Das erfindungsgemäße Verfahren wird bevorzugt bei einem Druck im Kolonnenkopf von 10 mbar bis 5 bar, bevorzugt von 10 bis 200 mbar, weiter bevorzugt 10 bis 50 mbar, durchgeführt.

Bevorzugt ist im oberen Kolonnenbereich eine Temperaturregelung vorgesehen, mit einer Messstelle unterhalb der obersten theoretischen Trennstufe, bevorzugt auf der dritten theoretischen Trennstufe von oben gezählt, die als Stellgröße den Destillatstrom, das Rücklaufverhältnis oder bevorzugt die Rücklaufmenge, nutzt. Dadurch wird ein stabiler Betrieb der Kolonne gewährleistet mit der Folge einer weiteren Verbesserung der erzielbaren Produktreinheit.

In einer weiteren Verfahrensvariante ist zusätzlich oder alternativ eine Temperaturregelung im unteren Kolonnenbereich vorgesehen mit einer Messstelle oberhalb der untersten theoretischen Trennstufe, bevorzugt auf der zweiten theoretischen Trennstufe von unten gezählt, die als Stellgröße die Sumpfentnahmemenge nutzt. Durch diese zusätzliche Maßnahme wird eine weitere Verbesserung des stabilen Kolonnenbetriebs erreicht.

Die Kolonne weist eine Anzahl von theoretischen Trennstufen von 4 bis 50, bevorzugt von 5 bis 20 auf.

Der seitliche Zulauf für das Gemisch enthaltend Cyclohexyl(meth)acrylat (das Roh-Cyclohexyl(meth)acrylat) erfolgt im Bereich beginnend mindestens eine theoretisch Trennstufe oberhalb der untersten und endend mindestens eine theoretische Trennstufe unterhalb der obersten theoretischen Trennstufe.

Bei einer Trennstufenzahl von ≥ 40 bis 50 befindet sich diese Zulaufstelle bevorzugt 5 bis 25 theoretische Trennstufen oberhalb der untersten und 5 bis 25 theoretische Trennstufen unterhalb der obersten Stufe.

Bei einer Trennstufenzahl von ≥ 10 bis 20 befindet sich diese Zulaufstelle bevorzugt 3 bis 10 theoretische Trennstufen oberhalb der untersten und 3 bis 10 theoretische Trennstufen unterhalb der obersten Stufe.

Z.B. ist die Zulaufstelle für das von Roh-Cyclohexyl(meth)acrylat bei einer Kolonne mit 45 bis 50 Trennstufen bevorzugt auf der theoretischen Trennstufe zwischen der 4. bis 41. Trennstufe, und bei einer Kolonne mit 20 Trennstufen bevorzugt auf der theoretischen Trennstufe zwischen der 2. bis 18. theoretischen Trennstufe angeordnet.

Das der Rektifikationskolonne seitlich zugeführte Gemisch weist bevorzugt folgende Zusammensetzung auf:
50 bis 98 Gew.-%, besonders 75 bis 98 Gew.-%, z.B. 76 bis 98 Gew.-%, Cyclohexyl(meth)acrylat,
0,5 bis 20 Gew.-%, besonders 0,5 bis 10 Gew.-%, Cyclohexanol,
0,5 bis 20 Gew.-%, besonders 0,5 bis 8 Gew.-%, Schleppmittel,
0,25 bis 5 Gew.-%, besonders 0,25 bis 3 Gew.-%, Höhersieder [bezogen auf Cyclohexyl(meth)acrylat],
0,25 bis 5 Gew.-%, besonders 0,25 bis 3 Gew.-%, weitere Leichtersieder [bezogen auf Cyclohexyl(meth)acrylat].

Bezüglich der trennwirksamen Einbauten gibt es grundsätzlich keine Einschränkungen; bevorzugt sind Füllkörper, geordnete Packungen oder Böden vorgesehen.

In einer bevorzugten Ausgestaltung sind die Einbauten so zu wählen, dass sie einen spezifischen Druckverlust von 0,05 bis 10 mbar/theoretischer Trennstufe, bevorzugt 0,1 bis 5 mbar/theoretischer Trennstufe, bewirken.

Unter dem spezifischen Druckverlust versteht man das Verhältnis von Druckverlust der Einbauten und der theoretischen Trennstufenzahl.

In einer bevorzugten Verfahrensvariante werden Hochleistungsfüllkörper als trennwirksame Einbauten in der Kolonne eingesetzt.

Hochleistungsfüllkörper für Prozesse der chemischen und verwandten Industrie besitzen eine fluiddynamisch günstige Grundform mit hoher trennaktiver volumenbezogener Oberfläche.

Im Vergleich zu herkömmlichen Füllkörpern zeichnet sich ein Hochleistungsfüllkörper sowohl durch bessere fluiddynamische Eigenschaften als auch durch eine höhere Effektivität der Stoffübertragung aus. Er bietet einen extrem niedrigen Druckverlust bei bestem Stoffaustausch. Beispiele für solche Hochleistungsfüllkörper finden sich in Chemie Ingenieur Technik 2010, 82 No. 10, Seiten 1693 bis 1703, (R. Billet et al.; DOI: 10.1002/cite.201000050).

Bei der Destillationseinheit, in der das reine Cyclohexyl(meth)acrylat schließlich über Kopf oder einen Seitenablauf abgetrennt wird, handelt es sich bevorzugt um einen Fallfilmverdampfer, Dünschichtverdampfer oder eine Destillationskolonne.

Das im erfindungsgemäßen Verfahren anfallende reine Cyclohexylacrylat, gewonnen aus einem Gemisch enthaltend Cyclohexylacrylat, Cyclohexanol und Schleppmittel, weist besonders eine Reinheit von > 98 Gew.-%, ganz besonders ≥ 98,5 Gew.-%, weiter besonders ≥ 99 Gew.-%, auf.

Das im erfindungsgemäßen Verfahren anfallende reine Cyclohexylmethacrylat, gewonnen aus einem Gemisch enthaltend Cyclohexylmethacrylat, Cyclohexanol und Schleppmittel, weist besonders eine Reinheit von > 98 Gew.-%, ganz besonders ≥ 98,5 Gew.-%, weiter besonders ≥ 99 Gew.-%, auf.

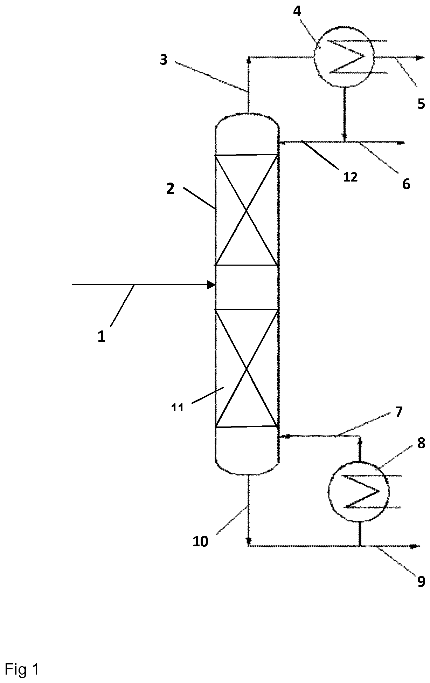

Die Erfindung wird im Folgenden anhand einer Zeichnung (Figur 1) und Ausführungsbeispielen näher erläutert.

Figur 1 zeigt die schematische Darstellung einer bevorzugten Rektifikationskolonne zur Durchführung des erfindungsgemäßen Verfahrens. Der Zulaufstrom wird über die Zulaufleitung 1 in die Kolonne 2 geführt.

Die Kolonne ist mit Füllkörpern oder Packungen gefüllt (11). Der am Kolonnenkopf anfallende Brüdenstrom 3 wird im Kondensator 4, der gegebenenfalls durch einen Nachkühler ergänzt wird, teilweise kondensiert und in den Rücklaufstrom 12 sowie den Destillatstrom 6 aufgeteilt. Der nicht kondensierte Anteil aus dem Kondensator 4 enthält die leichtsiedenden Verunreinigungen und wird dampfförmig als Strom 5 abgezogen. Am unteren Kolonnenende wird die Flüssigkeit 10 in einem Sumpfverdampfer 8 teilweise verdampft und über die Rohrleitung 7 in die Kolonne zurückgeführt. Ein Hauptstrom 9, der die schwersiedenden Verunreinigungen enthält, wird abgezogen. Der Verdampfer 8 kann als Naturumlaufverdampfer oder als Zwangsumlaufverdampfer ausgebildet sein, im letzteren Fall ist zusätzlich eine Umlaufpumpe für den Flüssigkeitsstrom 10 erforderlich. Besonders vorteilhaft hinsichtlich der Vermeidung unerwünschter Polymerisationsreaktionen ist es anstelle des Zwangsumlaufverdampfers einen Fallfilmverdampfer oder Dünnschichtverdampfer einzusetzen, da mit diesen Bauarten sehr kurze Verweilzeiten möglich sind.

Zur Verringerung der Verweilzeit der Flüssigkeit im Verdampfer ist es günstig, den Sumpfraum inkl. Sumpfleitung so zu gestalten, dass ein möglichst geringes Flüssigkeitsvolumen vorhanden ist.

Alle Druckangaben beziehen sich auf den Absolutdruck.

Ein 'Leichtersieder' [bezogen auf Cyclohexyl(meth)acrylat] ist ein Stoff, dessen Siedetemperatur niedriger als die Siedetemperatur von Cyclohexylacrylat bzw. Cyclohexylmethacrylat bei gleichem Druck ist.

Ein 'Höhersieder' [bezogen auf Cyclohexyl(meth)acrylat] ist ein Stoff, dessen Siedetemperatur höher als die Siedetemperatur von Cyclohexyl(meth)acrylat bzw. Cyclohexylmethacrylat bei gleichem Druck ist.

### Beispiele

### 1) Vergleichsbeispiel

Ein Roh-Cyclohexylmethacrylat von 883 kg/h mit einer Temperatur von 97 °C wurde am oberen Ende der Füllkörperschüttung mit Pall-Ringen 25 mm in der Kolonne aufgegeben.

Die Zusammensetzung des Roh-Cyclohexylmethacrylats wurde mit Hilfe von Analysen bestimmt:

| | |
|---|---|
| Cyclohexylmethacrylat | 95,2 Gew.-% |
| Cyclohexanol | 2,8 Gew.-% |
| Cyclohexan | 1,0 Gew.-% |
| weitere Leichtersieder, Höhersieder: | 1,0 Gew.-% |

Der Betrieb der Kolonne erfolgte bei einem Kopfdruck von 16 mbar und einem Sumpfdruck von 37 mbar. Am Kopf der Kolonne wurde das Destillat kondensiert und in einem Behälter gesammelt. Dieser wurde mittels Standregelung kontinuierlich abgelassen, sodass ein ständiger Rückführstrom von Destillat mit 134 kg/h in die erste Veresterungsstufe erfolgte.

Die Zusammensetzung des Destillates wurde mit Hilfe von Analysen bestimmt und betrug:

| | |
|---|---|
| Cyclohexylmethacrylat | 82 Gew.-% |
| Cyclohexanol | 11 Gew.-% |
| Cyclohexan | 5 Gew.-% |
| weitere Leichtersieder | 2 Gew.-% |

Der Sumpf der Kolonne durchläuft einen Fallfilmverdampfer der im Gegenstrom betrieben wird. Der Fallfilmverdampfer wurde dabei mit 93 kW beheizt. Das aus dem Fallfilmverdampfer auslaufende Sumpfprodukt von 746 kg/h hatte dabei eine Temperatur von 107 °C.

Die Zusammensetzung wurde mit Hilfe von Analysen bestimmt:

| | |
|---|---|
| Cyclohexylmethacrylat | 98 Gew.-% |
| Cyclohexanol | 1,3 Gew.-% |
| Höhersieder | 0,7 Gew.-% |

Die handelsüblichen Spezifikationen für Cyclohexylmethacrylat wurden mit 98 Gew.-% Cylcohexylmethacrylat eingehalten. Die Destillationsausbeute für Cyclohexylmethacrylat dieser Kolonne lag bei über 86 %.

### 2) Versuche zur Temperaturstabilität eines Zulaufproduktes (Seitenzulauf der Rektifikationskolonne)

Versuche zur Temperaturstabilität eines Zulaufproduktes, hier eines Gemisches aus
94,2 Gew.-% Cyclohexylmethacrylat,
2,5 Gew.-% Cyclohexanol, 1,0 Gew.-% Cyclohexen,
1,4 Gew.-% Höhersieder und 0,9 Gew.-% weitere Leichtersieder,
bei Normaldruck, drei verschiedenen Temperaturen (120, 130, 140 °C) und einer Verweilzeit von einer Stunde haben gezeigt, dass die Bildung von Dimeren und Oligomeren, die für die Verschlechterung der Produktqualität des Zielesters verantwortlich sind, mit zunehmender Temperatur ansteigt.

Im Folgenden ist die Erhöhung des Cyclohexylmethacrylat-Dimeren der Formel 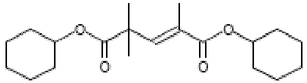 durch Temperaturerhöhung dargestellt (gaschromatographische Analyse):

| | |
|---|---|
| Ausgangsmaterial: | 0,52 Flächenprozent |
| 120 °C | 0,56 Flächenprozent |
| 130 °C | 0,64 Flächenprozent |
| 140 °C | 0,69 Flächenprozent |

### 3) Erfindungsgemäßes Beispiel

Die Fahrweise wird anhand der Daten aus einer thermodynamischen Simulation einer Gesamtanlage zur Herstellung von Cyclohexylmethacrylat dargestellt.

Die thermodynamische Simulation des Prozesses wurde mit der Software Aspen Plus® (kurz: Aspen) durchgeführt. Aspen ist eine umfangreiche Simulationssoftware, die zur Modellierung, Simulation und Optimierung chemischer Verfahren und Anlagen in der Industrie eingesetzt wird. Aspen verfügt über umfangreiche Modell-Datenbanken zur Modellierung der Basisoperationen sowie über Stoffdatenbanken für die Stoffeigenschaften vieler verschiedener Substanzen. Die Eigenschaften von Mischungen werden von Aspen mit Hilfe von unterschiedlichen thermodynamischen Modellen aus den Stoffdaten der reinen Substanzen berechnet.

Die thermodynamische Simulation der Gesamtanlage führte zu folgenden Ergebnissen:
Ein Roh-Cyclohexylmethacrylat von 883 kg/h mit einer Temperatur von 97 °C wird auf der 5. theoretischen Trennstufe in eine Rektifikationskolonne mit 12 theoretischen Trennstufen aufgegeben.

Die Zusammensetzung des zulaufenden Roh-Cyclohexylmethacrylate beträgt:

| | |
|---|---|
| Cyclohexylmethacrylat | 94,7 Gew.-% |
| Cyclohexanol | 2,7 Gew.-% |
| Cyclohexan | 1 Gew.-% |
| weitere Leichtersieder | 0,7 Gew.-% |
| Höhersieder | 0,9 Gew.-% |

Der Betrieb der Kolonne erfolgt bei einem Kopfdruck von 16 mbar und einem Sumpfdruck von 37 mbar. Der spezifische Druckverlust der Kolonne beträgt 1,75 mbar/theoretischer Trennstufe. Die Kopftemperatur beträgt 61 °C. Am Kopf der Kolonne wird das Destillat kondensiert. Der Rückführstrom von Destillat in die erste Veresterungsstufe beträgt 40 kg/h. Die Kolonne wird bei einem Rücklaufverhältnis von 3,1 betrieben.

Die Zusammensetzung des Destillates beträgt:

| | |
|---|---|
| Cyclohexylmethacrylat | 10 Gew.-% |
| Cyclohexanol | 59 Gew.-% |
| Cyclohexan | 15 Gew.-% |
| weitere Leichtersieder | 16 Gew.-% |

Der Verdampfer wird dabei mit 31 kW betrieben. Das aus dem Verdampfer auslaufende Sumpfprodukt von 840 kg/h hat dabei eine Temperatur von 107 °C.

Die Zusammensetzung des Sumpfablaufs beträgt:

| | |
|---|---|
| Cyclohexylmethacrylat | 99 Gew.-% |
| Cyclohexanol | 0,1 Gew.-% |
| Höhersieder | 0,9 Gew.-% |

Durch das erfindungsgemäße Verfahren kann die Destillation von Roh-Cylcohexyl(meth)acrylat bei gleicher Tageskapazität von z.B. 14,0 t unter Einhaltung der geforderten Spezifikationen mit einer Energiekosteneinsparung von 69 % gegenüber einem herkömmlichen Destillationsverfahren durchgeführt werden.

Durch die Reduzierung der Rückführung zur Veresterung von 134 kg/h auf 40 kg/h kann die Tageskapazität einer Gesamtanlage zur Herstellung von Cyclohexyl(meth)acrylat z.B. um ca. 100 kg/h gesteigert werden, das entspricht einer Kapazitätssteigerung von ca. 14 %.

Durch die Erniedrigung der Sumpftemperatur im Verdampfer von 113 °C auf 107 °C wird die Bildung von unbekannten Nebenkomponenten, die für das Fouling im Verdampfer verantwortlich sind, erheblich reduziert.

Da die Laufzeit einer Gesamtanlage zur Herstellung von Cyclohexyl(meth)acrylat durch die Laufzeit der Verdampfer aufgrund von Fouling limitiert ist, kann durch die Verringerung der Nebenkomponentenbildung durch die reduzierte Sumpftemperatur in der Leichtsiederdestillation die Laufzeit einer Gesamtanlage gesteigert werden. Dies führt zu einer höheren Jahreskapazität der Gesamtanlage.

## Patentansprüche

1. Verfahren zur destillativen Gewinnung von reinem Cyclohexyl(meth)acrylat aus einem Gemisch enthaltend Cyclohexyl(meth)acrylat, Cyclohexanol und Schleppmittel, **dadurch gekennzeichnet, dass** man das Gemisch einer Rektifikationskolonne, mit Sumpfverdampfer, trennwirksamen Einbauten und einem Abtriebs- und einem Verstärkungsteil, als Seitenzulauf zuführt, wobei die Kolonne 4 bis 50 theoretische Trennstufen aufweist, der seitliche Zulauf im Bereich beginnend mindestens eine Trennstufe oberhalb der untersten und endend mindestens eine Trennstufe unterhalb der obersten Stufe erfolgt, der Druck am Kopf der Kolonne im Bereich von 10 mbar bis 5 bar liegt und das Rücklaufverhältnis 1 : 0,2 bis 1 : 10 beträgt, und den Sumpfaustrag einer weiteren Destillationseinheit zuführt, in der das Cyclohexyl(meth)acrylat über Kopf oder einen Seitenablauf abgetrennt wird.

2. Verfahren nach Anspruch 1, **dadurch gekennzeichnet, dass** es sich bei dem Schleppmittel um Cyclohexan handelt.

3. Verfahren nach einem der vorhergehenden Ansprüche, **dadurch gekennzeichnet, dass** es sich bei den trennwirksamen Einbauten um Füllkörper, geordnete Packungen oder Böden handelt.

4. Verfahren nach dem vorhergehenden Anspruch, **dadurch gekennzeichnet, dass** die trennwirksamen Einbauten einen spezifischen Druckverlust im Bereich von 0,05 bis 10 mbar pro theoretischer Trennstufe bewirken.

5. Verfahren nach einem der beiden vorhergehenden Ansprüche, **dadurch gekennzeichnet, dass** es sich bei den Füllkörpern um Hochleistungsfüllkörper handelt.

6. Verfahren nach einem der vorhergehenden Ansprüche, **dadurch gekennzeichnet, dass** die Verweilzeit im Sumpfverdampfer und dem zugehörigen Rohrleitungssystem im Bereich von 1 bis 60 Minuten beträgt.

7. Verfahren nach einem der vorhergehenden Ansprüche 1 bis 5, **dadurch gekennzeichnet, dass** die Verweilzeit im Sumpfverdampfer und dem zugehörigen Rohrleitungssystem im Bereich von 10 bis 30 Minuten beträgt.

8. Verfahren nach einem der vorhergehenden Ansprüche, **dadurch gekennzeichnet, dass** es sich bei dem Sumpfverdampfer um einen Dünnschichtverdampfer oder Fallfilmverdampfer handelt.

9. Verfahren nach einem der vorhergehenden Ansprüche, **dadurch gekennzeichnet, dass** im Sumpfverdampfer die Verdampfung bei einer spezifischen Wärmebelastung im Bereich von 1 bis 100 kW/m² erfolgt.

10. Verfahren nach einem der vorhergehenden Ansprüche 1 bis 8, **dadurch gekennzeichnet, dass** im Sumpfverdampfer die Verdampfung bei einer spezifischen Wärmebelastung im Bereich von 2 bis 50 kW/m² erfolgt.

11. Verfahren nach einem der vorhergehenden Ansprüche 1 bis 8, **dadurch gekennzeichnet, dass** im Sumpfverdampfer die Verdampfung bei einer spezifischen Wärmebelastung im Bereich von 5 bis 30 kW/m² erfolgt.

12. Verfahren nach einem der vorhergehenden Ansprüche, **dadurch gekennzeichnet, dass** die Kolonne 5 bis 20 theoretische Trennstufen aufweist und der seitliche Zulauf im Bereich beginnend mindestens eine Trennstufe oberhalb der untersten und endend mindestens eine Trennstufe unterhalb der obersten Stufe erfolgt.

13. Verfahren nach einem der vorhergehenden Ansprüche, **dadurch gekennzeichnet, dass** der Druck am Kopf der Kolonne im Bereich von 10 mbar bis 200 mbar liegt.

14. Verfahren nach einem der vorhergehenden Ansprüche, **dadurch gekennzeichnet, dass** das Rücklaufverhältnis 1 : 0,2 bis 1 : 1 beträgt.

15. Verfahren nach einem der vorhergehenden Ansprüche, **dadurch gekennzeichnet, dass** das der Rektifikationskolonne seitlich zugeführte Gemisch folgende Zusammensetzung aufweist:
50 bis 98 Gew.-% Cyclohexyl(meth)acrylat,
0,5 bis 20 Gew.-% Cyclohexanol,
0,5 bis 20 Gew.-% Schleppmittel,
0,25 bis 5 Gew.-% Höhersieder [bezogen auf Cyclohexyl(meth)acrylat],
0,25 bis 5 Gew.-% weitere Leichtersieder [bezogen auf Cyclohexyl(meth)acrylat].

16. Verfahren nach einem der vorhergehenden Ansprüche zur Gewinnung von reinem Cyclohexylacrylat, aus einem Gemisch enthaltend Cyclohexylacrylat, Cyclohexanol und Schleppmittel, mit einer Reinheit von > 98 Gew.-%.

17. Verfahren nach einem der vorhergehenden Ansprüche zur Gewinnung von reinem Cyclohexylmethacrylat, aus einem Gemisch enthaltend Cyclohexylmethacrylat, Cyclohexanol und Schleppmittel, mit einer Reinheit von > 98 Gew.-%.

## Claims

1. A process for isolating pure cyclohexyl (meth)acrylate from a mixture comprising cyclohexyl (meth)acrylate, cyclohexanol and entrainer by distillation, wherein the mixture is fed as side feed stream into a rectification column having a bottom vaporizer, separation-active internals and a stripping section and an enrichment section, where the column has from 4 to 50 theoretical plates, the side feed stream is introduced in the region commencing at least one theoretical plate above the bottommost theoretical plate and ending at least one theoretical plate below the uppermost theoretical plate, the pressure at the top of the column is in the range from 10 mbar to 5 bar and the reflux ratio is from 1:0.2 to 1:10, and the bottom output is fed to a further distillation unit in which the cyclohexyl (meth)acrylate is separated off at the top or at a side offtake.

2. The process according to claim 1, wherein the entrainer is cyclohexane.

3. The process according to either of the preceding claims, wherein the separation-active internals are random packing elements, ordered packing or trays.

4. The process according to the preceding claim, wherein the separation-active internals bring about a specific pressure drop in the range from 0.05 to 10 mbar per theoretical plate.

5. The process according to either of the two preceding claims, wherein the random packing elements are high-performance random packing elements.

6. The process according to any of the preceding claims, wherein the residence time in the bottom vaporizer and the associated piping system is in the range from 1 to 60 minutes.

7. The process according to any of preceding claims 1 to 5, wherein the residence time in the bottom vaporizer and the associated piping system is in the range from 10 to 30 minutes.

8. The process according to any of the preceding claims, wherein the bottom vaporizer is a thin film evaporator or falling film evaporator.

9. The process according to any of the preceding claims, wherein the vaporization in the bottom vaporizer is effected at a specific heat input in the range from 1 to 100 kW/m².

10. The process according to any of preceding claims 1 to 8, wherein the vaporization in the bottom vaporizer is effected at a specific heat input in the range from 2 to 50 kW/m².

11. The process according to any of preceding claims 1 to 8, wherein the vaporization in the bottom vaporizer is effected at a specific heat input in the range from 5 to 30 kW/m².

12. The process according to any of the preceding claims, wherein the column has from 5 to 20 theoretical plates and the side feed stream is introduced in the region commencing at least one theoretical plate above the bottommost theoretical plate and ending at least one theoretical plate below the uppermost theoretical plate.

13. The process according to any of the preceding claims, wherein the pressure at the top of the column is in the range from 10 mbar to 200 mbar.

14. The process according to any of the preceding claims, wherein the reflux ratio is from 1:0.2 to 1:1.

15. The process according to any of the preceding claims, wherein the mixture fed in at the side of the rectification column has the following composition:
from 50 to 98% by weight of cyclohexyl (meth)acrylate,
from 0.5 to 20% by weight of cyclohexanol,
from 0.5 to 20% by weight of entrainer,
from 0.25 to 5% by weight of relatively high boilers [relative to cyclohexyl (meth)acrylate],
from 0.25 to 5% by weight of further relatively low boilers [relative to cyclohexyl (meth)acrylate].

16. The process according to any of the preceding claims for isolating pure cyclohexyl acrylate having a purity of > 98% by weight from a mixture comprising cyclohexyl acrylate, cyclohexanol and entrainer.

17. The process according to any of the preceding claims for isolating pure cyclohexyl methacrylate having a purity of > 98% by weight from a mixture comprising cyclohexyl methacrylate, cyclohexanol and entrainer.

## Revendications

1. Procédé d'obtention par distillation de (méth)acrylate de cyclohexyle pur à partir d'un mélange contenant du (méth)acrylate de cyclohexyle, du cyclohexanol et des agents d'entraînement, **caractérisé en ce que** le mélange est introduit dans une colonne de rectification, munie d'un évaporateur de fond, de composants à activité de séparation et d'une partie de rectification et d'enrichissement, en tant qu'alimentation latérale, la colonne comprenant 4 à 50 étapes de séparation théoriques, l'alimentation latérale ayant lieu dans la zone débutant au moins une étape de séparation au-dessus de l'étape la plus inférieure et terminant au moins une étape de séparation en dessous de l'étape la plus supérieure, la pression à la tête de la colonne se situant dans la plage allant de 10 mbar à 5 bar, et le taux de reflux étant de 1:0,2 à 1:10, et la sortie de fond étant introduite dans une unité de distillation supplémentaire, dans laquelle le (méth)acrylate de cyclohexyle est séparé par la tête ou une évacuation latérale.

2. Procédé selon la revendication 1, **caractérisé en ce que** l'agent d'entraînement consiste en du cyclohexane.

3. Procédé selon l'une quelconque des revendications précédentes, **caractérisé en ce que** les composants à activité de séparation consistent en des corps de remplissage, des garnissages ordonnés ou des plateaux.

4. Procédé selon la revendication précédente, **caractérisé en ce que** les composants à activité de séparation produisent une perte de pression spécifique dans la plage allant de 0,05 à 10 mbar par étape de séparation théorique.

5. Procédé selon l'une quelconque des deux revendications précédentes, **caractérisé en ce que** les corps de remplissage consistent en des corps de remplissage haute performance.

6. Procédé selon l'une quelconque des revendications précédentes, **caractérisé en ce que** le temps de séjour dans l'évaporateur de fond et le système de canalisation correspondant est dans la plage allant de 1 à 60 minutes.

7. Procédé selon l'une quelconque des revendications 1 à 5 précédentes, **caractérisé en ce que** le temps de séjour dans l'évaporateur de fond et le système de canalisation correspondant est dans la plage allant de 10 à 30 minutes.

8. Procédé selon l'une quelconque des revendications précédentes, **caractérisé en ce que** l'évaporateur de fond consiste en un évaporateur à couche mince ou un évaporateur à film tombant.

9. Procédé selon l'une quelconque des revendications précédentes, **caractérisé en ce que** l'évaporation dans l'évaporateur de fond a lieu à une charge thermique spécifique dans la plage allant de 1 à 100 kW/m².

10. Procédé selon l'une quelconque des revendications 1 à 8 précédentes, **caractérisé en ce que** l'évaporation dans l'évaporateur de fond a lieu à une charge thermique spécifique dans la plage allant de 2 à 50 kW/m².

11. Procédé selon l'une quelconque des revendications 1 à 8 précédentes, **caractérisé en ce que** l'évaporation dans l'évaporateur de fond a lieu à une charge thermique spécifique dans la plage allant de 5 à 30 kW/m².

12. Procédé selon l'une quelconque des revendications précédentes, **caractérisé en ce que** la colonne comprend 5 à 20 étapes de séparation théoriques et l'alimentation latérale a lieu dans la zone débutant au moins une étape de séparation au-dessus de l'étape la plus inférieure et terminant au moins une étape de séparation en dessous de l'étape la plus supérieure.

13. Procédé selon l'une quelconque des revendications précédentes, **caractérisé en ce que** la pression à la tête de la colonne se situe dans la plage allant de 10 mbar à 200 mbar.

14. Procédé selon l'une quelconque des revendications précédentes, **caractérisé en ce que** le taux de reflux est de 1:0,2 à 1:1.

15. Procédé selon l'une quelconque des revendications précédentes, **caractérisé en ce que** le mélange introduit latéralement dans la colonne de rectification présente la composition suivante :
50 à 98 % en poids de (méth)acrylate de cyclohexyle,
0,5 à 20 % en poids de cyclohexanol,
0,5 à 20 % en poids d'agent d'entraînement,
0,25 à 5 % en poids de composants de point d'ébullition élevé [par rapport au (méth)acrylate de cyclohexyle],
0,25 à 5 % en poids de composants de point d'ébullition faible supplémentaires [par rapport au (méth)acrylate de cyclohexyle].

16. Procédé selon l'une quelconque des revendications précédentes pour l'obtention d'acrylate de cyclohexyle pur, à partir d'un mélange contenant de l'acrylate de cyclohexyle, du cyclohexanol et des agents d'entraînement, avec une pureté > 98 % en poids.

17. Procédé selon l'une quelconque des revendications précédentes pour l'obtention de méthacrylate de cyclohexyle pur, à partir d'un mélange contenant du méthacrylate de cyclohexyle, du cyclohexanol et des agents d'entraînement, avec une pureté > 98 % en poids.
